# EUROPEAN PATENT SPECIFICATION

(11) **EP 1 683 514 B1**
(45) Date of publication and mention of the grant of the patent: **23.05.2012**
(21) Application number: 05075156.9
(22) Date of filing: 20.01.2005
(51) Int. Cl.: A61K 9/72, A61K 9/14

(54) **Process for the preparation of concentrated suspensions of drugs and kit thereof**
Verfahren zur Herstellung konzentrierter Suspensionen und Kit davon
Procédé de préparation de suspensions concentrées et une trousse de ceci

(43) Date of publication of application: 26.07.2006
(73) Proprietor: Eratech S.r.l., 20122 Milano (IT)
(72) Inventor: Caponetti, Giovanni, 29100 Piacenza (IT); Artusi, Mariella, 43100 Parma (IT)
(74) Representative: Zanoli, Enrico

(56) References cited:
- WO-A-00/25746
- WO-A-02/41925
- WO-A-99/18971
- WO-A-03/086347
- WO-A-2004/054545
- "Operating instructions of SUCELL" In: Anonymous: "System-Partikel-Technik", Sympatec GmbH pages 1-2,

## Description

The present invention relates to concentrated suspensions of water insoluble pharmaceutical substances, to their preparation process and their use to prepare therapeutically acceptable suspensions ready for inhalatory administration.

An effective inhalation therapy requires that particles of any drugs are delivered to the lower airways surfaces, particularly in the deep lung region, which is the site of drug action where the main pharmacological effects occur. To achieve delivery in the deep lung region, the size of the drug particles must be such as to avoid early deposition in the upper airways. As it is well known, the respiratory tract acts as a filter of the inhaled particles, which are captured and deposited depending on their size. Drug particles which ensure optimal deposition have a size in the range between 0.1 and 10 µm, preferably between 0.5 and 7 µm.

Although widely used, suspensions of pharmaceutical substances may be difficult to prepare, particularly with respect to their physical stability. A drawback of such suspensions is the tendency of the particles to agglomerate and form a precipitate that settles at rest over time. Suitable surfactants and salts usually assist in re-dispersing the settled particles by gentle shaking, so that a homogeneous suspension is re-established. When such sediment of settled particles is not easily broken or the particles cannot be easily re-dispersed since have undergone coagulation, a risk exists that a wrong dose of substance is administered to a patient, either in excess or in defect.

A drug to be administered as an inhalatory suspension must be micronized to cut down the size to less than 10 µm. Such size reduction, typically performed in a dry state, may lead to an initial aggregation of drug particles. Thus, the corresponding suspension for inhalatory administration is prepared by suspending the drug in water by means of a high energy mixer, or often by a turboemulsifier.

WO 00/25746 discloses a process for the preparation of inhalatory suspensions by dispersing micronized drug particles in water in a turboemulsifier provided with magnetic stirring, optionally followed by treatment in a high-pressure homogenizer. The operating conditions adopted require the turboemulsifier to be operated at a speed comprised between 2500 and 2600 rpm, for a time of 15-20 minutes. The size of the particles of a suspension produced by this method, however, does not appear to be improved over the conventional inhalatory suspensions, the size distribution being as follows: d(0.1) = 0.76 µm; d(0.5) = 3.01 µm; d(0.9) = 9.42 µm, with a calculated span value of [d(0.9) - d(0.1)]/d(0.5) = 2.88. Implementation of a subsequent dispersion step in a high-pressure homogenizer at a pressure as high as 1000 bar generated an inhalatory suspension with the following size distribution: d(0.1) = 0.82 µm; d(0.5) = 2.43 µm; d(0.9) = 5.07 µm, with a calculated span value of [d(0.9) - d(0.1)]/d(0.5) = 1.75.

These results show that the process described in WO 00/25746 brings about a slight reduction of about 0.6 µm in d(0.5), a more meaningful reduction of about 4 µm in d(0.9) and an improvement of the span only when a dispersion step in a high pressure homogenizer is implemented after a dispersion step in a turboemulsifier. It appears that such a high pressure treatment is required to achieve a size reduction of the coarser fraction as indicated by the d(0.9) values, thereby making the process burdensome and economically less attractive.

WO 2004/054545 (Example 2) discloses the preparation of sterile suspensions for nebulisation comprising the preparation of an aqueous phase containing pharmaceutically acceptable excipients, then the preparation of a suspension by precipitating a pharmaceutical substance from an organic solvent in said aqueous solution. The crude suspension so obtained is then treated in a high-pressure homogenizer up to 600 bar.

WO 99/18971 (Example 2 and 3) discloses the preparation of suspensions of mometasone furoate by separate sterilization of the drug and of the aqueous medium. Also, the suspension is then treated in a microfluidizer at a pressure of about 1206 bar (17500 psi).

WO 03/086347D discloses to sterilize a pharmaceutical substance as described in WO00/25746, discussed above. Example 2 describes the preparation of a suspension that includes a sterilization of the liquid medium (water + sodium chloride + surfactant) by thermal treatment at 121°C in a turboemulsifier for 20 minutes, followed by the addition of a pre-sterilized BDP (described in Example 1).

WO 02 41 925 discloses a method of sterilising a pharmaceutical composition by rapidly heating the composition, and diluting a sterile concentrate under sterile conditions.

With respect to sterilization of aqueous suspensions of pharmaceutical substances, particularly of glucocorticosteroids, it is reported in WO 99/25359 that attempts at terminal sterilization of such suspensions have all proved unsatisfactory.

It would therefore be desirable to provide a process to prepare suspensions of pharmaceutical substances for inhalatory administration which is efficient, economically attractive and capable of producing stable suspensions over the time, so that a desired drug dose can be administered to a patient.

It would also be desirable to provide a process to effectively sterilize drug particles in suspension with satisfactory results.

Further, it would be desirable to provide inhalatory suspensions of drug particles that can be prepared extemporaneously, namely just before administration.

According to a first aspect, the invention provides a process to prepare a concentrated suspension of water insoluble pharmaceutical substances, characterized by comprising the following steps:
a) dissolving at least one pharmaceutically acceptable surfactant in water to form an aqueous solution of said surfactant;
b) suspending in said aqueous solution micronized particles of said water insoluble pharmaceutical substance with a particle size distribution in which the d(0.5) fraction is less than 3 µm and the span value is less than 2, at a concentration between 5 and 50 times the concentration of said pharmaceutical substance in a therapeutically acceptable suspension for inhalatory administration;
c) dissolving a salt in said aqueous solution, at a concentration that: i) does not cause coagulation of said particles of pharmaceutical substance; and ii) generates upon dilution an inhalatory suspension in which the concentration of salt in the aqueous phase is not higher than 10 g/l,
said steps being carried out under stirring and at a pressure not higher than 2 bar.

A second aspect of the invention provides a kit for the extemporaneous preparation of a suspension of water insoluble pharmaceutical substances intended for inhalatory administration, characterized by comprising and a separate diluent in proportions such that a β or γ irradiated concentrated suspension of water insoluble pharmaceutical substances, characterized by comprising:
a) micronized particles of at least one water insoluble pharmaceutical substance having:
   i) a particle size distribution in which the d(0.5) fraction is less than 4 µm, the d(0.9) fraction is less than 9 µm, the span value is less than 2.5 upon aging the suspension for 90 days;
   ii) a reduction in total purity of the active material of less than 0.5% from the initial purity of the untreated material;
   said pharmaceutical substance being present at a concentration of between 0.5 and 100 g/l;
b) an aqueous solution of a pharmaceutically acceptable surfactant as dispersing medium for said micronized particles of pharmaceutical substance;
c) a salt dissolved in said aqueous solution, at a concentration that: i) does not cause coagulation of said particles of pharmaceutical substance; and ii) generates upon dilution an inhalatory suspension in which the concentration of salt in the aqueous phase is not higher than 10 g/l,
upon mixing said diluent with said concentrated suspension, a suspension ready for inhalatory administration is obtained, in which said pharmaceutical substance is present at a concentration of from 0.1 to 2.0 g/l.

With the expression "pharmaceutical substance" it is a meant a substance exerting a biological activity, typically for therapy, prophylaxis or diagnosis purposes.

With the expression "substance insoluble in water" it is meant a substance having a solubility in water of less than 10 g/l, usually of less than 5 g/l, and even of less than 1 g/l.

Pharmaceutical substances suitable to be administered as inhalatory suspensions are *β*2-receptors active substances, glucocorticoids, other antiallergic or anti-inflammatory substances or derivatives thereof, although the invention is not limited to them.

Suitable *β*2-receptors active substances are terbutaline, salbutamol, mabuterol, fenoterol, formoterol, orciprenaline, isoprenaline, isoetharine, clenbuterol, hexoprenaline, procaterol, 1-(4-hydroxyphenyl)-2-[1,1-dimethyl-3-(2― methoxyphenyl) propylamino]-ethanol, 1-(3,5-dihydroxyphenyl)-2-[1, 1-dimethyl-3-(2-methoxyphenyl) propylamino] -ethanol, 1-3, 4-dihydroxyphenyl )-2-[1, 1-dimethyl-3-(2-methoxyphenyl) propylamino] -ethanol, (4-hydroxy-α'-[[[6-(4-phenylbutoxy) -hexyl] -amino] -methyl]-1,3 benzyldimetha-nol, pharmacologically acceptable salts or derivatives thereof and compounds of similar pharmacological properties.

Suitable glucocorticoids are compounds like budesonide, beclomethasone, dexamethasone, flumethasone, flunisolide, fluticasone, triamcinolone acetonide, 17- and/or 21-esters of these steroids, pharmacologically acceptable salts thereof and compounds of similar pharmacological properties.

Preferred pharmaceutical substances according to the invention are budesonide, beclomethasone dipropionate (BDP), mometasone furoate, fluticasone propionate their derivatives and similar glucocorticoids with anti-inflammatory activity.

A suspension ready for inhalatory administration, or inhalatory suspension, as intended in the present application, is a suspension of one or more pharmaceutical substance insoluble in water at a concentration between 0.1 and 1.5 g/l.

Typical inhalatory suspensions of BDP have a concentration of 0.4 g/l; typical inhalatory suspensions of budesonide have a concentration of 0.25 or 0.5 g/l; typical inhalatory suspensions of fluticasone have a concentration of 0.25 or 1.00 g/l.

As mentioned above, physical stability over the time is a critical aspect of suspensions pharmaceutical substances. Burdensome production process is another critical aspect.

Also disclosed is a concentrated non-inhalatory suspension of water insoluble pharmaceutical substances that is relatively simple to produce, is stable over the time, allows easy storage, transportation and handling, in particular effective sterilization, and can be used to prepare inhalatory suspensions with the required particle size distribution by simple dilution, either at a production site or directly before administration (extemporaneous preparation).

By "micronization" in the present context is meant a process involving mechanical means, for instance milling or grinding, to reduce the particle size to the micrometer range. Information about micronization can be found e.g. in "The Theory and Practice of Industrial Pharmacy", Lachman, Liebermann and Klang, 2^{nd} Ed., Lea & Febiger, Philadelphia, USA.

Upon aging of 90 days, the micronized particles of the water insoluble pharmaceutical substance in the concentrated non inhalatory suspension of the invention are characterized by a particle size distribution in which the d(0.5) fraction is less than 3, the d(0.9) fraction is less than 9 µm and the span value is less than 2. Preferably, the d(0.9) fraction is less than 7 µm. Such distribution ensures good stability during storage and is designed to generate by simple dilution an inhalatory suspension with optimal drug delivery in the lower airways surfaces.

Furthermore, the concentration of water insoluble pharmaceutical substance in the concentrated non inhalatory suspension varies from 5 to 50 times the concentration of such substance in a suspension for inhalatory administration. Since this latter concentration is not higher than 2 g/l, the maximum concentration of the pharmaceutical substance in the concentrated non inhalatory suspension is 100 times this value, namely 200 g/l. Preferably, the concentration of the pharmaceutical substance in the concentrated non inhalatory suspension is comprised between 0.2 : 150 g/l, the preferred concentration range of the same substance in the inhalatory suspension being 0.1:1.5 g/l. More preferably the concentration of the pharmaceutical substance in the concentrated non inhalatory suspension is comprised between 0.5 : 10 g/l, even more preferably between 1 and 8 g/l.

The surfactant dissolved in the aqueous phase of the suspension of the invention can be any pharmaceutically acceptable surfactant. Suitable surfactants are nonionic or cationic surfactants. Preferred are nonionic surfactants like polysorbates and block copolymers of polyoxyethylene and polyoxypropylene, known as "Poloxamers". Polysorbates are described in the CTFA International Cosmetic Ingredient Dictionary as mixtures of esters of fatty acids of sorbitol and sorbitol anhydride condensed with ethylene oxide, commercially known as "Span". Particularly preferred are non ionic surfactant of the family known as "Tween", more particularly those surfactants known as "Tween" and "Tween 80", a commercial polyoxyetylensorbitan mono-oleate.

A surfactant as described above is present in the concentrated suspension of the invention in an amount up to the limit of solubility in the aqueous phase.

Isotonic solution or normal saline contains 9 g/l of salt. Inhalatory suspensions of the invention are isotonic or about isotonic, but can also be hypotonic or slightly hypertonic, the overall salt concentration ranging from 4 to 10 g/l of salt. Preferred salt is sodium chloride.

The concentration of salt in the concentrated suspension according to the invention must not be as high to cause coagulation of the particles due to lowering or elimination of the repulsive barrier among suspended particles, known also as "salting out". This means that, depending on the case, formation of an isotonic solution in the inhalatory suspension by dilution of the concentrated non inhalatory suspension may require addition of salt during dilution to avoid salting out of the concentrated suspension.

The concentrated non-inhalatory suspension of water insoluble pharmaceutical substances may contain other components, such as pH buffers, preservatives and excipients, as is known the field.

The concentrated non-inhalatory suspension of water insoluble pharmaceutical substances can be prepared by first dissolving a pharmaceutically acceptable surfactant in water to form a rather concentrated solution of surfactant.

Then the micronized particles of the water insoluble pharmaceutical substance are slowly added to such solution under stirring. This step is carried out in a small volume of water, to ensure effective coating of the particles by the surfactant. When a cationic surfactant is used, interaction between particles of pharmaceutical substance and surfactant is promoted by controlling the pH of the solution. Possible heat generated during dissolution can promote adsorption of surfactant on the surface of the particles. A high energy mixer equipped with turbine having a small holes grid (emulsions type) is used at a speed of 7,000 rpm for about 10 minutes.

Then a salt is added, preferably NaCl, which reduces the repulsive forces among the particles and stabilizes the suspension, possibly causing settling of particles which in any event can be easily re-dispersed by gentle shaking. Such salt also ensures, or contributes to ensure, that a solution with the desired salt concentration is formed by dilution of this concentrated suspension.

This concentrated non inhalatory suspension of pharmaceutical substances is subjected to sterilization by *β* irradiation preferably ranging from 2 to 10 KGy or by *γ* irradiation preferably ranging from 2 to 8 KGy. It has been found that the sterilization carried out on the concentrated suspension is fully satisfactory, since a dramatic reduction of the microbial count was achieved. Also, the particle size distribution of the sterilized suspension did not vary significantly after sterilization and upon 90 days of aging. It has also been found that no significant degradation of the pharmaceutical substance was caused by irradiation, as shown by the high purity of substance after irradiation.

Simple dilution upon stirring or gentle shaking of the concentrated non inhalatory suspension of the water insoluble pharmaceutical substances according to the invention brings about the formation of a ready to use, or extemporaneous, inhalatory suspension. Dilution is carried out with water or an aqueous solution of salt, depending on whether dilution with water only generates a normal saline or a slightly hypertonic or hypotonic solution, as described above, or whether a salt make-up is required. Dilution can be carried out in conventional mixers at a speed of not more than 500 rpm.

It has been found that the inhalatory suspension so prepared retains substantially the particle size distribution of the concentrated non inhalatory suspension, even upon aging, as well as physical stability.

When a sterilized concentrated non inhalatory suspension is used, a corresponding inhalatory suspension can be produced by diluting the concentrated suspension in a sterile room. This allows to carry out sterilization on small volumes of concentrated suspensions to generate industrial batches of sterile substances, avoiding terminal sterilization.

As an alternative, an inhalatory suspension of pharmaceutical substances can be prepared by dilution of the concentrated non inhalatory suspension directly before use, e.g. by a patient. To this purpose, there is provided a kit of components comprising a metered volume of said concentrated suspension and a metered volume of water or aqueous solution of a salt, kept in separate containers or in separate compartments of the same container, intended to be put into contact to form an inhalatory suspension having the desired concentration of the components. Such inhalatory suspension contains one or more pharmacological unit of the desired substance ensuring appropriate dosage level.

Treatment and handling of concentrated suspensions as opposed to diluted, inhalatory suspensions, brings about other process advantages that are apparent to a skilled person, including possibility to handle substances in selected and controlled areas of plants or laboratories and to treat such suspensions under laminar flow hoods.

### EXAMPLES

### Reference Examples 1- 4

### Preparation of concentrated non-inhalatory suspensions of Beclomethasone Dipropionate (BDP)

Table 1 shows the composition of the suspension for each example. Ex. 1 is a comparative example (no salt).

**TABLE 1**

| **Components** | **Amount (g/l)** | | | |
|---|---|---|---|---|
| **Example** | **1 (comp.)** | **2** | **3** | **4** |
| Beclomethasone Dipropionate | 2.0 | 2.0 | 2.0 | 4.0 |
| SPAN 20 | 1.0 | 1.0 | 1.0 | 2.0 |
| TWEEN 20 | 5.0 | 5.0 | 5.0 | 10.0 |
| Sodium Chloride | - | 9.0 | 4.2 | 9.0 |
| Distilled Water | Suff. to 11 | Suff. to 11 | suff. to 11 | suff. to 11 |
| Concentration vs inhalatory suspension | x5 | x5 | x5 | x10 |

The surfactants TWEEN 20 and SPAN 20 were added to distilled water and mixed for 5 minutes until complete dissolution at a speed of 1600 rpm in a mixer with a turbine model Silverson L4RT equipped with a standard mixing head, at room temperature.

Micronized Beclomethasone Dipropionate (BDP) powder was added slowly under stirring the solution so obtained. The micronized powder had the following particle size distribution: d(0.1) 0.44 µm, d(0.5) 1.13 µm e d(0.9) 2.42 µm.

The BDP was then homogenized in the water solution of the two surfactants for 15 minutes at the speed of 7000 rpm by means of a mixer with a turbine model Silverson L4RT equipped with standard mixing head and operating at room temperature.

In examples 2, 3 and 4 sodium chloride was added to the concentrated suspensions, under stirring.

With the method of preparation described above non-inhalatory suspensions have been obtained in which the concentration of BDP is 5 or 10 times (as shown in the last line of Table 1) the concentration of a commercial inhalatory suspension of BDP (0.4 mg/ml).

The particle size distribution of such concentrated suspensions was then determined with a Particle Size Analizer Sympatec immediately after preparation (time t = 0) and upon aging of 7, 14 and 90 days (t=7; t=14; t=90). The span value is calculated with the formula Span = [d(0.9) - d(0.1)]/d(0.5), given before in this specification.

The results obtained are shown in Table 2.

**Table 2**

| **Ex** | **Particle Size Distribution (PSD)** | | | | | | | | | | | | | | | |
|---|---|---|---|---|---|---|---|---|---|---|---|---|---|---|---|---|
| | **t = 0** | | | | **t = 7** | | | | **t = 14** | | | | **t = 90** | | | |
| | **d_{0.1}** | **d_{0.5}** | **d_{0.9}** | **span** | **d_{0.1}** | **d_{0.5}** | **d_{0.9}** | **span** | **d_{0.1}** | **d_{0.5}** | **d_{0.9}** | **span** | **d_{0.1}** | **d_{0.5}** | **d_{0.9}** | **span** |
| **1** | 1.03 | 2.39 | 5.18 | 1.74 | 1.11 | 2.60 | 5.23 | 1.58 | 1.11 | 2.70 | 5.40 | 1.59 | 1.29 | 4.12 | 10.68 | 2.27 |
| **2** | 1.09 | 2.54 | 5.58 | 1.76 | 1.11 | 2.58 | 5.36 | 1.65 | 1.09 | 2.58 | 5.49 | 1.70 | 1.10 | 2.74 | 5.89 | 1.74 |
| **3** | 1.09 | 2.47 | 5.15 | 1.64 | 1.11 | 2.62 | 5.51 | 1.68 | 1.11 | 2.60 | 5.44 | 1.67 | 1.07 | 2.50 | 5.16 | 1.63 |
| **4** | 1.12 | 2.74 | 6.66 | 2.02 | 1.13 | 2.78 | 6.07 | 1.77 | 1.09 | 2.70 | 5.71 | 1.71 | 1.17 | 2.95 | 6.14 | 1.68 |

It appears from the data of Table 2 that the PSD of example 2, 3 and 4 do not vary significantly over the time, although a settling of solid particles occurs, upon aging of 90 days. The suspension of Comparative Example 1, however, undergoes a significant increase of the d(0.5) and d(0.9) fractions after 90 days. In example 2, 3 and 4 the initial PSD is re-established by simple manual shaking of the suspension. This demonstrates that the micronized pharmaceutical substance is dispersed in the water solution in a stable way.

### Reference Examples 1a - 4a

### Preparation of inhalatory BDP suspensions from the concentrated suspensions.

The concentrated non inhalatory suspensions containing BDP described in examples 1 to 4 were diluted to form inhalatory suspensions 1a - 4a ready to be administered.

The diluent was distilled water in the case of example 1a, and distilled water containing sodium chloride in the case of the other examples. The amount of sodium chloride in the diluent solution was such as to yield a concentration of 0.9% p/v (isotonic) in example 2a and 4a, and a concentration of 0.42% p/v (hypotonic) in the case of example 3a. The PSD of the diluted inhalatory suspensions was determined immediately after their preparation (t= 0). The results are shown in Table 3. The PSD shows that the suspensions are suitable for inhalatory administration.

**Table 3**

| **Example** | **PSD @ t= 0** | | | |
|---|---|---|---|---|
| | **d_{0.1}** | **d_{0.5}** | **d_{0.9}** | **span** |
| **1a** | 1.08 | 2.46 | 5.09 | 1.61 |
| **2a** | 1.07 | 2.48 | 5.21 | 1.66 |
| **3a** | 1.08 | 2.51 | 5.44 | 1.73 |
| **4a** | 1.11 | 2.71 | 5.99 | 1.80 |

### Reference Examples 5 -10

### Preparation of concentrated non-inhalatory suspensions of Budesonide

Table 4 shows the composition of the suspension for each example. Ex. 5 is a comparative example (no salt).

**Table 4**

| **Components** | **Amount (g/l)** | | | | | |
|---|---|---|---|---|---|---|
| **Example** | **5 (comp.)** | **6** | **7** | **8** | **9** | **10** |
| Budesonide | 1.25 | 1.25 | 1.25 | 2.5 | 5.0 | 5.0 |
| TWEEN 20 | 1.0 | 1.0 | 1.0 | 1.0 | 1.0 | 0.5 |
| Sodium chloride | - | 4.2 | 9.0 | 9.0 | 9.0 | 9.0 |
| Distilled Water | Suff. to 11 | Suff. To 11 | Suff. to 11 | suff. to 11 | suff. to 11 | suff. to 11 |
| Conc. vs inhalatory susp. | x 5 | x 5 | x 5 | x 5 | x 20 | x 20 |

The surfactant TWEEN 80 was added to distilled water and mixed for 5 minutes until complete dissolution at a speed of 1600 rpm in a mixer with a turbine model Silverson L4RT equipped with a standard mixing head, at room temperature.

Micronized Budesonide powder was added slowly under stirring the solution so obtained. The micronized powder had the following particle size distribution: d(0.1) 0.50 µm, d(0.5) 1.39µm; d(0.9) 3.15 µm.

The budesonide was then homogenized in the water solution of the surfactant for 15 minutes at the speed of 7000 rpm by means of a mixer with turbine model Silverson L4RT equipped with standard mixing head and operating at room temperature.

In example 6, 7, 8, 9 and 10 sodium chloride was added to the concentrated suspensions, under stirring, in the amount shown.

With the method of preparation described above non-inhalatory suspensions have been obtained in which the concentration of budesonide is 5 or 20 times (as shown in the last line of Table 4) the concentration of a commercial inhalatory suspension of budesonide (0.25 or 0.5 mg/ml).

The particle size distribution of such concentrated suspensions was then determined with a Particle Size Analizer Sympatec immediately after preparation (time t = 0) and upon aging of 7, 14 and 90 days (t=7; t=14; t=90). The span value is calculated with the formula given before in this specification.

The results obtained are shown in Table 5.

**Table 5**

| **Ex** | **Particle Size Distribution (PSD)** | | | | | | | | | | | | | | | |
|---|---|---|---|---|---|---|---|---|---|---|---|---|---|---|---|---|
| | **t = 0** | | | | **t = 7** | | | | **t = 14** | | | | **t = 90** | | | |
| | **d_{0.1}** | **d_{0.5}** | **d_{0.9}** | **span** | **d_{0.1}** | **d_{0.5}** | **d_{0.9}** | **span** | **d_{0.1}** | **d_{0.5}** | **d_{0.9}** | **span** | **d_{0.1}** | **d_{0.5}** | **d_{0.9}** | **span** |
| **5** | 0.78 | 1.75 | 3.53 | 1.57 | 0.82 | 1.82 | 3.99 | 1.74 | 0.80 | 1.77 | 3.98 | 1.80 | 0.80 | 1.79 | 4.40 | 2.01 |
| **6** | 0.79 | 1.76 | 3.55 | 1.57 | 0.81 | 1.77 | 3.48 | 1.51 | 0.83 | 1.93 | 3.89 | 1.58 | 0.78 | 1.73 | 3.47 | 1.55 |
| **7** | 0.79 | 1.84 | 3.68 | 1.57 | 0.80 | 1.81 | 3.59 | 1.54 | 0.80 | 1.73 | 3.35 | 1.47 | 0.79 | 1.75 | 3.50 | 1.55 |
| **8** | 0.81 | 1.74 | 3.45 | 1.52 | 0.79 | 1.81 | 3.60 | 1.55 | NA | NA | NA | NA | 0.81 | 1.98 | 4.06 | 1.64 |
| **9** | 0.77 | 1.75 | 3.48 | 1.55 | 0.79 | 1.86 | 3.78 | 1.61 | 0.80 | 1.81 | 3.62 | 1.56 | 0.78 | 1.77 | 3.53 | 1.55 |
| **10** | 0.77 | 1.75 | 3.48 | 1.55 | 0.79 | 1.79 | 3.56 | 1.55 | 0.80 | 1.77 | 3.48 | 1.51 | 0.76 | 1.76 | 3.52 | 1.57 |

It appears from the data of Table 5 that the PSD of example 6, 7, 8, 9 and 10 do not vary significantly over the time, although a settling of solid particles occurs, even upon aging of 90 days. The suspension of Comparative Example 5 undergoes an increase of the d(0.9) fraction after 90 days. In the other examples the initial PSD is re-established by simple manual shaking of the suspension. This demonstrates that the micronized pharmaceutical substance is dispersed in the water solution in a stable way.

### Reference Examples 5a - 10a

### Preparation of inhalatory suspensions of budesonide from the concentrated suspensions.

The concentrated non inhalatory suspensions containing budesonide described in examples 5 to 10 were diluted to form inhalatory suspensions 5a - 10a ready to be administered, containing 0.25 or 0.5 mg/ml of budesonide.

The diluent was distilled water in the case of example 5a, and a water solution of sodium chloride in the other examples. The amount of sodium chloride in the diluent solution was such as to yield a concentration of 0.9% p/v (isotonic) or a concentration of 0.42% p/v (hypotonic), depending on the example. The PSD of the diluted inhalatory suspensions was determined immediately after their preparation (t= 0). For the suspension of example 8a, 9a and 10a the PSD was determined also after aging of 90 days. The results are shown in Table 6. The PSD shows that the suspensions are suitable for inhalatory administration.

**Table 6**

| **Example** | **PSD** | | | | | | | |
|---|---|---|---|---|---|---|---|---|
| | **t = 0** | | | | **t= 90** | | | |
| | **d_{0.1}** | **d_{0.5}** | **d_{0.9}** | **span** | **d_{0.1}** | **d_{0.5}** | **d_{0.9}** | **span** |
| **5a** | 0.80 | 1.74 | 3.41 | 1.50 | NA | NA | NA | NA |
| **6a** | 0.81 | 1.76 | 3.56 | 1.56 | NA | NA | NA | NA |
| **7a** | 0.81 | 1.75 | 3.54 | 1.56 | NA | NA | NA | NA |
| **8a** | 0.80 | 1.78 | 3.54 | 1.54 | 0.80 | 1.88 | 3.75 | 1.57 |
| **9a** | 0.84 | 1.77 | 3.54 | 1.47 | 0.78 | 1.72 | 3.49 | 1.57 |
| **10a** | 0.79 | 1.81 | 3.53 | 1.51 | 0.79 | 1.76 | 3.61 | 1.60 |

### Example 11 (comparative)

### Preparation of a suspension of BDP (0.4 mg/ml) containing NaCl 0.9% according to WO 00/25746

Purpose of this comparative example is to prepare directly an inhalatory suspension of beclomethasone dipropionate in aqueous solution of sodium chloride and Tween 20 surfactant with the same concentration of the composition of example 2 of WO 00/25746, then determine the PSD of the suspension so obtained and compare it with the PSD of inhalatory suspensions of the inventions, which are prepared from concentrated suspensions in turn prepared using micronized BDP with the same particle size distribution. This shows whether the preparation process involving an intermediate production of concentrated suspensions of the invention improves the quality of the final PSD.

450 mg of Tween 20, 90 mg of Span 20 and 3.65 g of NaCl were put in a vessel with 450 ml of distilled water and mixed under magnetic stirring for 20 minutes until complete dissolution of the two components.

180 mg of BDP micronized powder were added to the solution so prepared. PSD of the powder was: d(0.1) 0.44 µm, d(0.5) 1.13 µm e d(0.9) 2.42 µm, namely the same PSD of the BDP powder used to prepare the concentrated suspensions of examples 1 - 4.

The BDP powder was then homogenized in the water solution of the two surfactants for 20 minutes at the speed of 2600 rpm. The BDP concentration was of 0.4 mg/ml.

The suspension obtained was analyzed to determine its PSD by means of a Particle Size Analizer Sympatec.

The results are shown in Table 7.

**Table 7**

| **Ex. 11** | **Particle Size Distribution** | | | | | | | | | | | |
|---|---|---|---|---|---|---|---|---|---|---|---|---|
| | **t=0** | | | | **T = 21** | | | | **t=90** | | | |
| | **d _{0.1}** | **d _{0.5}** | **d_{0.9}** | **span** | **d_{0.1}** | **d_{0.5}** | **d_{0.9}** | **span** | **d_{0.1}** | **d_{0.5}** | **d_{0.9}** | **span** |
| | 1.19 | 5.70 | 14.23 | 2.28 | 1.43 | 7.91 | 20.13 | 2.36 | 1.29 | 4.52 | 12.11 | 2.4 |

The PSD of the inhalatory suspension prepared with the process above shows a substantial increase of the size of the particles in each fraction, since the time 0. The increase of the d_{0.5} and of the d_{0.9} fraction is dramatic. This shows that inhalatory suspensions prepared directly from powders without high pressure homogenization do not produce powders with the desired particle size distribution.

### Example 12

### Sterilization of a x 5 concentrated non inhalatory suspension of budesonide by irradiation with β or γ rays.

The concentrated non inhalatory suspension of Example 8 was irradiated with *β* rays at a dose of 2,4,6,8 10 KGy; and with *γ* rays at a dose of 2,4,6,8 KGy.

The PSD of the suspension was then determined at t = 0, t = 45 and t = 90 days.

The results are shown in Table 8. A comparison PSD on a non-irradiated sample is shown at line 1.

**Table 8**

| **Example 12: Particle Size Distribution** | | | | | | | | | | | | |
|---|---|---|---|---|---|---|---|---|---|---|---|---|
| **Dose** | **t = 0** | | | | **t = 45** | | | | **t = 90** | | | |
| | **d_{0.1}** | **d_{0.5}** | **d_{0.9}** | **span** | **d_{0.1}** | **d_{0.5}** | **d_{0.9}** | **span** | **d_{0.1}** | **d_{0.5}** | **d_{0.9}** | **span** |
| **Comparison** | 0.81 | 1.74 | 3.45 | 1.52 | 0.80 | 1.76 | 3.51 | 1.54 | 0.81 | 1.98 | 4.06 | 1.64 |
| ***β* : 2 KGy** | 0.78 | 1.76 | 3.54 | 1.57 | 0.78 | 1.75 | 3.48 | 1.54 | 0.78 | 1.80 | 3.60 | 1.57 |
| ***β* : 4 KGy** | 0.79 | 1.87 | 3.82 | 1.62 | 0.78 | 1.81 | 3.65 | 1.58 | 0.78 | 1.77 | 3.52 | 1.55 |
| ***β* : 6 KGy** | 0.79 | 1.91 | 3.96 | 1.66 | 0.79 | 1.88 | 3.82 | 1.61 | 0.77 | 1.76 | 3.52 | 1.56 |
| ***β* : 8 KGy** | 0.78 | 1.86 | 3.81 | 1.63 | 0.85 | 2.09 | 4.25 | 1.63 | 0.86 | 2.20 | 4.61 | 1.70 |
| ***β* : 10 KGy** | 0.78 | 1.89 | 3.96 | 1.68 | 0.77 | 1.72 | 3.46 | 1.56 | 0.76 | 1.70 | 3.44 | 1.57 |
| ***γ* : 2 KGy** | 0.78 | 1.82 | 3.66 | 1.58 | 0.79 | 1.76 | 3.51 | 1.54 | 0.78 | 1.70 | 3.39 | 1.53 |
| ***γ* : 4 KGy** | 0.79 | 1.88 | 3.86 | 1.63 | 0.79 | 1.74 | 3.49 | 1.55 | 0.78 | 1.77 | 3.53 | 1.55 |
| ***γ* : 6 KGy** | 0.79 | 1.84 | 3.75 | 1.61 | 0.79 | 1.76 | 3.53 | 1.56 | 0.77 | 1.82 | 3.67 | 1.59 |
| ***γ* : 8 KGy** | 0.78 | 1.83 | 3.82 | 1.66 | 0.78 | 1.86 | 3.80 | 1.62 | 0.77 | 1.71 | 3.45 | 1.57 |

A test of microbial count was then carried out on the irradiated suspensions to determine if a satisfactory sterility was achieved. A purity test was also carried out to determine if any degradation of the pharmaceutical substance budesonide was provoked by the irradiation treatment.

As a comparison, a test of microbial count and purity was carrier out also on a sample of micronized budesonide powder.

The results are reported in Table 9 and Table 10 below.

**Table 9**

| **BETA IRRADIATION** | | | | |
|---|---|---|---|---|
| | **Budesonide Powder** | | **Concentrated Budesonide Suspension (x 5)** | |
| **Intensity of Irradiation (KGy)** | **Microbial count UFC/mg** | **Purity %** | **Microbial count UFC/ml** | **Purity %** |
| **0** | 13 | 99.15 | 3.5 · 10⁴ | 99.06 |
| **2** | Sterile | 99.15 | Sterile | 99.07 |
| **4** | Sterile | 99.08 | Sterile | 99.08 |
| **6** | Sterile | 99.05 | Sterile | 99.10 |
| **8** | Sterile | 98.97 | Sterile | 99.01 |
| **10** | Sterile | 98.95 | Sterile | 99.03 |

**Table 10**

| **GAMMA IRRADIATION** | | | | |
|---|---|---|---|---|
| | **Budesonide Powder** | | **Concentrated Budesonide Suspension (x 5)** | |
| **Intensity of Irradiation (KGy)** | **Microbial count UFC/mg** | **Purity %** | **Microbial count UFC/ml** | **Purity %** |
| **0** | 13 | 99.15 | 3.5 · 10⁴ | 99.06 |
| **2** | Sterile | 98.88 | Sterile | 98.97 |
| **4** | Sterile | 98.84 | Sterile | 98.91 |
| **6** | Sterile | 98.88 | Sterile | 98.98 |
| **8** | Sterile | 98.69 | Sterile | 98.91 |

### Example 12a

The concentrated non inhalatory suspension sterilized by treatment with *β* rays at 10 KGy was then diluted with distilled water to produce a correspondingly *β* irradiated inhalatory suspension with a concentration of budesonide of 0.5 mg/ml, containing also an additional amount of sodium chloride so that the concentration of sodium chloride in the diluted suspension is 0.9% p/v.

The concentrated non inhalatory suspension sterilized by treatment with *γ* rays at 8 KGy was then diluted with distilled water to produce a correspondingly *γ* irradiated inhalatory suspension with a concentration of budesonide of 0.5 mg/ml, containing also an additional amount of sodium chloride so that the concentration of sodium chloride in the diluted suspension is 0.9% p/v.

The PSD at the time 0 is shown in Table 11. The *β* or *γ* irradiated inhalatory suspensions obtained by dilution of the corresponding *β* or *γ* irradiated concentrated suspensions have not undergone any significant increase in the PSD.

**Table 11**

| **PSD** | | | | |
|---|---|---|---|---|
| **Example 12a** | **t=0** | | | |
| | **d_{0.1}** | **d_{0.5}** | **d_{0.9}** | **span** |
| **Comparison** | 0.80 | 1.78 | 3.54 | 1.54 |
| ***β*-10 KGy** | 0.78 | 1.80 | 3.70 | 1.62 |
| ***γ*-8 KGy** | 0.79 | 1.75 | 3.51 | 1.55 |

### Example 13

### Sterilization of a x 5 concentrated non inhalatory suspension of beclomethasone dipropionate by irradiation with β or γ rays.

The concentrated non inhalatory suspension of Example 2 was irradiated with *β* rays at a dose of 2,4,6,8 e 10 KGy; and with *γ* rays at a dose of 2,4,6,8 KGy.

The PSD of the suspension was then determined at t = 0, t = 45 and t = 90 days.

The results are shown in Table 12. A comparison PSD on a non-irradiated sample is shown at line 1.

**Table 12**

| **Example 13: Particle Size Distribution** | | | | | | | | | | | | |
|---|---|---|---|---|---|---|---|---|---|---|---|---|
| **Dose** | **t = 0** | | | | **t = 45** | | | | **t=90** | | | |
| | **d_{0.1}** | **d_{0.5}** | **d_{0.9}** | **span** | **d_{0.1}** | **d_{0.5}** | **d_{0.9}** | **span** | **d_{0.1}** | **d_{0.5}** | **d_{0.9}** | **span** |
| **Comparison** | 1.09 | 2.77 | 6.21 | 1.85 | 1.14 | 2.95 | 6.54 | 1.83 | 1.10 | 2.74 | 5.89 | 1.75 |
| ***β*: 2 KGy** | 1.13 | 3.04 | 7.14 | 1.98 | 1.14 | 2.95 | 6.75 | 1.90 | 1.14 | 3.15 | 8.09 | 2.20 |
| ***β*: 4 KGy** | 1.15 | 3.19 | 7.82 | 2.10 | 1.12 | 2.91 | 6.57 | 1.87 | 1.07 | 2.73 | 5.98 | 1.80 |
| ***β*: 6 KGy** | 1.13 | 3.03 | 7.03 | 1.95 | 1.12 | 2.88 | 6.48 | 1.86 | 1.08 | 2.73 | 5.97 | 1.76 |
| ***β*: 8 KGy** | 1.13 | 3.10 | 7.29 | 1.99 | 1.12 | 2.88 | 6.44 | 1.85 | 1.06 | 2.75 | 6.11 | 1.87 |
| ***β*: 10 KGy** | 1.12 | 3.00 | 6.98 | 1.95 | 1.12 | 2.90 | 6.52 | 1.86 | 1.08 | 2.76 | 6.11 | 1.82 |
| ***γ*: 2 KGy** | 1.14 | 3.06 | 7.07 | 1.94 | 1.12 | 2.89 | 6.49 | 1.86 | 1.07 | 2.76 | 6.26 | 1.88 |
| ***γ*: 4 KGy** | 1.15 | 3.06 | 7.09 | 1.94 | 1.14 | 2.94 | 6.68 | 1.88 | 1.07 | 2.75 | 6.16 | 1.85 |
| ***γ*: 6 KGy** | 1.14 | 3.04 | 7.07 | 1.95 | 1.12 | 2.93 | 6.74 | 1.91 | 1.08 | 2.76 | 6.16 | 1.84 |
| ***γ*: 8 KGy** | 1.13 | 2.98 | 6.98 | 1.96 | 1.11 | 2.89 | 6.65 | 1.91 | 1.09 | 2.76 | 6.11 | 1.82 |

A test of microbial count was then carried out on the irradiated suspensions to determine if a satisfactory sterility was achieved. A purity test was also carried out to determine if any degradation of the pharmaceutical substance BDP was provoked by the irradiation treatment.

As a comparison, a test of microbial count and purity was carrier out also on a sample of micronized BDP powder.

The results are reported in Table 13 and Table 14 below.

**Table 13**

| **BETA IRRADIATION** | | | | |
|---|---|---|---|---|
| | **BDP Powder** | | **Concentrated BDP Suspension (x 5)** | |
| **Intensity of irradiation (KGy)** | **Microbial count UFC/ mg** | **Purity %** | **Microbial count UFC/ml** | **Purity %** |
| **0** | 7 | 99.73 | 2.1 · 10³ | 99.70 |
| **2** | Sterile | 99.73 | Sterile | 99.74 |
| **4** | Sterile | 99.66 | Sterile | 99.64 |
| **6** | Sterile | 99.67 | Sterile | 99.67 |
| **8** | Sterile | 99.51 | Sterile | 99.56 |
| **10** | Sterile | 99.49 | Sterile | 99.60 |

**Table 14**

| **GAMMA IRRADIATION** | | | | |
|---|---|---|---|---|
| | **BDP Powder** | | **Concentrated BDP Suspension (x 5)** | |
| **Intensity of irradiation (KGy)** | **Microbial count UFC/ mg** | **Purity %** | **Microbial count UFC/ml** | **Purity %** |
| **0** | 7 | 99.73 | 2.1 · 10³ | 99.70 |
| **2** | Sterile | 99.73 | Sterile | 99.80 |
| **4** | Sterile | 99.68 | Sterile | 99.75 |
| **6** | Sterile | 99.70 | Sterile | 99.74 |
| **8** | Sterile | 99.07 | Sterile | 99.62 |

### Example 13a

The concentrated non inhalatory suspension sterilized by treatment with *β* rays at 10 KGy was then diluted with distilled water to produce a correspondingly *β* irradiated inhalatory suspension with a concentration of BDP of 0.4 mg/ml, containing also an additional amount of sodium chloride so that the concentration of sodium chloride in the diluted suspension is 0.9% p/v.

The concentrated non inhalatory suspension sterilized by treatment with *γ* rays at 8 KGy was then diluted with distilled water to produce a correspondingly *γ* irradiated inhalatory suspension with a concentration of BDP of 0.4 mg/ml, containing also an additional amount of sodium chloride so that the concentration of sodium chloride in the diluted suspension is 0.9% p/v.

The PSD at the time 0 is shown in Table 15. The *β* or *γ* irradiated inhalatory suspensions obtained by dilution of the corresponding *β* or *γ* irradiated concentrated suspensions have not undergone any significant increase in the PSD.

**Table 15**

| **PSD** | | | | |
|---|---|---|---|---|
| **Example 13a** | **t=0** | | | |
| | **d_{0.1}** | **d_{0.5}** | **d_{0.9}** | **span** |
| **Comparison** | 1.13 | 2.92 | 6.50 | 1.84 |
| ***β* -10 KGy** | 1.14 | 3.08 | 7.18 | 1.96 |
| ***γ* -8 KGy** | 1.11 | 2.95 | 6.91 | 1.97 |

## Claims

1. Kit for the extemporaneous preparation of a suspension of pharmaceutical substances having a solubility in water of less than 10 g/l, intended for inhalatory administration, **characterized by** comprising a β or γ irradiated concentrated suspension of pharmaceutical substances having a solubility in water of less than 10 g/l, **characterized by** comprising:
a) micronized particles of at least one water insoluble pharmaceutical substance having:
i) a particle size distribution in which the d(0.5) fraction is less than 4 µm, the d(0.9) fraction is less than 9 µm, the span value calculated with the formula [d(0.9) - d(0.1)]/d(0.5) is less than 2.5 upon aging the suspension of 90 days, said pharmaceutical substance being present at a concentration of between 0.5 and 100 g/l;
ii) a reduction in total purity of the active material of less than 0.5% from the initial purity of the untreated material;
b) an aqueous solution of a pharmaceutically acceptable surfactant as dispersing medium for said micronized particles of pharmaceutical substance;
c) a salt dissolved in said aqueous solution, at a concentration that: i) does not cause coagulation of said particles of pharmaceutical substance; and ii) generates upon dilution an inhalatory suspension in which the concentration of salt in the aqueous phase is not higher than 10g/l.
, and a separate diluent in proportions such that upon mixing said diluent with said β or γ irradiated concentrated suspension, a suspension ready for inhalatory administration is obtained, in which said pharmaceutical substance is present at a concentration of from 0.1 to 2.0 g/l.

2. Kit according to claim 1, wherein said pharmaceutical substance is a glucocorticoid.

3. Kit according to claim 2, wherein said glucocorticoid is selected from the group consisting of budesonide, beclomethasone, dexamethasone, flumethasone, flunisolide, fluticasone, triamcinolone acetonide, their pharmacologically acceptable 17 and/or 21 esters or salts and mometasone furoate.

4. Process for the preparation of a concentrated suspension of pharmaceutical substances having a solubility in water of less than 10 g/l, **characterized by** comprising the following steps:
a) dissolving a pharmaceutically acceptable surfactant in water to form an aqueous solution of said surfactant;
b) suspending in said aqueous solution micronized particles of said pharmaceutical substance with a particle size distribution in which the d(0.5) fraction is less than 3 µm and the span value calculated with the formula [d(0.9) - d(0.1)]/d(0.5) is less than 2, at a concentration between 5 and 50 times the concentration of said pharmaceutical substance in a therapeutically acceptable suspension for inhalatory administration having a concentration between 0.1 and 1.5 g/l;
c) dissolving a salt in said aqueous solution, at a concentration that: i) does not cause coagulation of said particles of pharmaceutical substance; and ii) generates upon dilution an inhalatory suspension in which the concentration of salt in the aqueous phase is not higher than 10 g/l ;
said steps being carried out under stirring and at a pressure not higher than 2 bar; and further comprising a sterilization step by subjecting said concentrated suspension to β or γ irradiation.

5. Process according to claim 4, **characterized in that** said sterilization step is carried out by means of β irradiation at a dose of from 2 to 10 KGy.

6. Process according to claim 4, wherein said irradiation is a γ irradiation at a dose of from 2 to 8 KGy.

## Patentansprüche

1. Kit für eine auf Rezept erfolgende Zubereitung einer Suspension pharmazeutischer Substanzen mit einer Löslichkeit in Wasser von weniger als 10 g/l zur Verabreichung durch Inhalation, **dadurch gekennzeichnet, dass** er aufweist: eine mit β- oder γ-Strahlen behandelte konzentrierte Suspension pharmazeutischer Substanzen mit einer Löslichkeit in Wasser von weniger als 10 g/l, die **dadurch gekennzeichnet ist, dass** sie aufweist:
a) mikronisierte Teilchen mindestens einer in Wasser unlöslichen pharmazeutischen Substanz mit:
i) einer Teilchengrößenverteilung, bei der die d(0,5)-Fraktion kleiner ist als 4 µm, die d(0,9)-Fraktion kleiner ist als 9 µm, der Spannenwert, der anhand der Formel [d(0,9) - d(0,1)] / d(0,5) berechnet wird, nach einer 90-tägigen Alterung der Suspension kleiner ist als 2,5, wobei die pharmazeutische Substanz in einer Konzentration zwischen 0,5 und 100 g/l vorhanden ist;
ii) einer Verringerung der Reinheit des aktiven Materials von insgesamt weniger als 0,5 % gegenüber der zu Anfang gegebenen Reinheit des unbehandelten Materials;
b) eine wässrige Lösung eines pharmazeutisch annehmbaren Tensids als Dispersionsmedium für die mikronisierten Teilchen der pharmazeutischen Substanz;
c) ein Salz, das in der wässrigen Lösung gelöst ist, und zwar mit einer Konzentration, die:
i) keine Koagulierung der Teilchen der pharmazeutischen Substanz bewirkt; und
ii) bei Verdünnung eine zur Inhalation geeignete Suspension erzeugt, in der die Salzkonzentration in der wässrigen Phase nicht höher ist als 10 g/l,
und ein separates Verdünnungsmittel in solchen Anteilen, dass beim Mischen des Verdünnungsmittels mit der mit β- oder γ-Strahlen behandelten konzentrierten Suspension eine Suspension erhalten wird, die zur Verabreichung durch Inhalation bereit ist und in der die pharmazeutische Substanz in einer Konzentration von 0,1 bis 2,0 g/l vorliegt.

2. Kit nach Anspruch 1, wobei die pharmazeutische Substanz Glucocorticoid ist.

3. Kit nach Anspruch 2, wobei das Glucocorticoid ausgewählt ist aus der Gruppe bestehend aus Budesonid, Beclomethason, Dexamethason, Flumethason, Flunisolid, Fluticason, Triamcinolonacetonid, ihren pharmazeutisch ausgewählten Estern oder Salzen und Mometasonfuroat.

4. Verfahren zum Herstellen einer konzentrierten Suspension pharmazeutisch annehmbarer Substanzen mit einer Löslichkeit in Wasser von weniger als 10 g/l, **dadurch gekennzeichnet, dass** es die folgenden Schritte umfasst:
a) Lösen eines pharmazeutisch annehmbaren Tensids in Wasser, um eine wässrige Lösung des Tensids zu bilden;
b) Suspendieren von mikronisierten Teilchen der pharmazeutischen Substanz mit einer Teilchengrößenverteilung, bei der die d(0,5)-Fraktion kleiner als 3 µm ist und der Spannenwert, der anhand der Formel [d(0,9) - d(0,1)] / d(0,5) berechnet wird, kleiner als 2 ist, mit einer Konzentration zwischen dem 5- bis 50-Fachen der Konzentration der pharmazeutischen Substanz in einer therapeutisch annehmbaren Suspension zur Verabreichung durch Inhalation, die eine Konzentration zwischen 0,1 und 1,5 g/l aufweist;
c) Lösen eines Salzes in der wässrigen Lösung bei einer Konzentration, die:
i) keine Koagulation der Teilchen der pharmazeutischen Substanz bewirkt; und
ii) ii) beim Verdünnen eine zur Inhalation geeignete Suspension bildet, bei der die Salzkonzentration in der wässrigen Phase nicht höher ist als 10 g/l;
wobei diese Schritte unter Rühren und bei einem Druck von nicht über 2 Bar ausgeführt werden;
und ferner einen Sterilisationsschritt umfasst, in dem die konzentrierte Suspension mit β- oder γ-Strahlen behandelt wird.

5. Verfahren nach Anspruch 4, **dadurch gekennzeichnet, dass** der Sterilisationsschritt anhand einer β-Bestrahlung bei einer Dosis von 2 bis 10 kGy durchgeführt wird.

6. Verfahren nach Anspruch 4, wobei die Bestrahlung eine γ-Bestrahlung bei einer Dosis von 2 bis 8 kGy ist.

## Revendications

1. Kit de préparation extemporanée d'une suspension de substances pharmaceutiques présentant une solubilité dans l'eau inférieure à 10 g/l, destinée à une administration par inhalation, **caractérisé en ce qu'**il comprend une suspension concentrée irradiée par rayonnement β ou γ de substances pharmaceutiques présentant une solubilité dans l'eau inférieure à 10 g/l **caractérisé en ce qu'**elle comprend :
a) des particules micronisées d'au moins une substance pharmaceutique insoluble dans l'eau présentant :
i) une distribution granulométrique selon laquelle la fraction d(0,5) est inférieure à 4 µm, la fraction d(0,9) est inférieure à 9 µm, l'intervalle de valeur calculé avec la formule [d(0,9)-d(0,1)]/d(0,5) est inférieur à 2,5 après vieillissement de la suspension pendant 90 jours, ladite substance pharmaceutique étant présente en une concentration comprise entre 0,5 et 100 g/l ;
ii) une réduction de la pureté totale de la matière active inférieure à 0,5% par rapport à la pureté initiale du matériau non traité ;
b) une solution aqueuse d'un agent tensioactif pharmaceutiquement acceptable agissant en tant que milieu de dispersion pour lesdites particules micronisées de substance pharmaceutique ;
c) un sel dissous dans ladite solution aqueuse à une concentration :
i) qui ne provoque pas la coagulation desdites particules de substance pharmaceutique ; et
ii) qui génère après dilution une suspension d'inhalation dans laquelle la concentration du sel dans la phase aqueuse n'est pas supérieure à 10 g/l ;
et un diluant distinct dans des proportions telles qu'après mélange dudit diluant avec ladite suspension concentrée irradiée par rayonnement β ou γ, soit obtenue une suspension prête à être utilisée pour une administration par inhalation, dans laquelle ladite substance pharmaceutique est présente à une concentration de 0,1 à 2,0 g/l.

2. Kit selon la revendication 1, dans lequel ladite substance pharmaceutique est un glucocorticoïde.

3. Kit selon la revendication 2, dans lequel ledit glucocorticoïde est choisi dans le groupe consistant en du budesonide, beclométhasone, dexaméthasone, fluméthasone, flunisolide, fluticasone, triamcinolone acétonide, leurs sels ou 17- et/ou 21-esters pharmaceutiquement acceptables, et du furoate de mométasone.

4. Procédé pour la préparation d'une suspension concentrée de substances pharmaceutiques présentant une solubilité dans l'eau inférieure à 10 g/l, **caractérisé en ce qu'**il comprend les étapes suivantes :
a) dissolution d'un agent tensioactif pharmaceutiquement acceptable dans de l'eau pour former une solution aqueuse dudit agent tensioactif ;
b) suspension dans ladite solution aqueuse de particules micronisées de ladite substance pharmaceutique présentant une distribution granulométrique selon laquelle la fraction d(0,5) est inférieure à 3 µm et l'intervalle de valeur calculé avec la formule [d(09)-d(0,1)]/d(0,5) est inférieur à 2, à une concentration comprise entre 5 et 50 fois la concentration de ladite substance pharmaceutique dans une suspension thérapeutiquement acceptable pour une administration par inhalation présentant une concentration comprise entre 0,1 et 1,5 g/l ;
c) dissolution d'un sel dans ladite solution aqueuse à une concentration :
i) qui ne provoque pas la coagulation desdites particules de substance pharmaceutique ; et
ii) qui génère après dilution une suspension d'inhalation dans laquelle la concentration en sel dans la phase aqueuse n'est pas supérieure à 10 g/l;
lesdites étapes étant mises en oeuvre sous agitation et à une pression non supérieure à 2 bars ;
et comprenant en outre une étape de stérilisation consistant à soumettre ladite suspension concentrée à une irradiation par rayonnement β ou γ.

5. Procédé selon la revendication 4, **caractérisé en ce que** ladite étape de stérilisation est réalisée par irradiation par rayonnement β à une dose de 2 à 10 KGy.

6. Procédé selon la revendication 4, dans lequel ladite irradiation est une irradiation par rayonnement γ à une dose de 2 à 8 KGy.
